(19) Europäisches Patentamt
European Patent Office
Office européen des brevets

(11) Veröffentlichungsnummer: **0 184 990**
**A1**

# (12) EUROPÄISCHE PATENTANMELDUNG

(21) Anmeldenummer: **85810556.2**

(22) Anmeldetag: **21.11.85**

(51) Int. Cl.⁴: **C 07 D 493/04**, A 61 K 31/35

(30) Priorität: **26.11.84 CH 5630/84**

(43) Veröffentlichungstag der Anmeldung: **18.06.86 Patentblatt 86/25**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Zimmermann, Markus, Dr., Steinbrecheweg 8, CH-4125 Riehhen (CH)**
Erfinder: **Wehrli, Walter, Dr., Aescherstrasse 23, CH-4054 Basel (CH)**
Erfinder: **Schmidtruppin, Karl Heinz, Dr., Im Baumgarten 5, CH-4144 Arlesheim (CH)**

(54) **Alkylidendioxy-Verbindungen.**

(57) Verbindungen der Formel

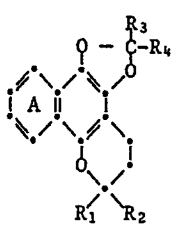

(I),

worin $R_1$ und $R_2$ unabhängig voneinander Wasserstoff, Niederalkyl oder Aryl oder zusammengenommen Niederalkylen bedeuten, $R_3$ und $R_4$ unabhängig voneinander Wasserstoff, Niederalkyl oder Aryl oder zusammengenommen Niederalkylen bedeuten, und der Ring A keine zusätzliche Substituenten hat oder zusätzlich durch Niederalkyl, Niederalkoxy und/oder Halogen substituiert ist, sind wirksam bei der durch einen Retrovirus induzierten Rauscher Leukämie der Maus und können daher therapeutisch beispielsweise bei der Bekämpfung von ebenfalls durch Retroviren verursachten Immunkrankheiten oder Autoimmunkrankheiten verwendet werden.

EP 0 184 990 A1

0184990

CIBA-GEIGY AG
Basel (Schweiz)

4-15168/+

# Alkylidendioxy-Verbindungen

Die vorliegende Erfindung betrifft Alkylidendioxy-Verbindungen sowie deren Herstellung, ferner pharmazeutische Präparate enthaltend solche Verbindungen und die Verwendung von letzteren als pharmakologische Wirkstoffe.

In erster Linie betrifft die vorliegende Erfindung die Verbindungen der Formel

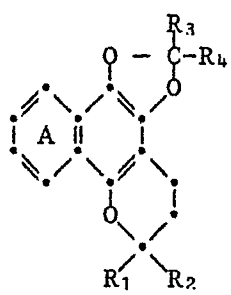

(I),

worin $R_1$ und $R_2$ unabhängig voneinander Wasserstoff, Niederalkyl oder Aryl oder zusammengenommen Niederalkylen bedeuten, $R_3$ und $R_4$ unabhängig voneinander Wasserstoff, Niederalkyl oder Aryl oder zusammengenommen Niederalkylen bedeuten, und der Ring A keine weiteren Substituenten hat oder zusätzlich durch Niederalkyl, Niederalkoxy und/oder Halogen substituiert ist.

Vorstehend, wie nachfolgend mit "nieder" bezeichnete monovalente organische Reste sowie Verbindungen enthalten bis einschliesslich 7, vorzugsweise bis einschliesslich 4, und in erster Linie 1 oder 2 Kohlenstoffatome. Bivalente organische Reste enthalten vorzugsweise 4 bis 6 Kohlenstoffatome.

Niederalkyl bedeutet in erster Linie Methyl und Aethyl, kann aber auch für n-Propyl, Isopropyl, n-Butyl, Isobutyl oder tert-Butyl, ferner für n-Pentyl, n-Hexyl und n-Heptyl stehen.

Aryl ist in erster Linie monocyclisches, carbocyclisches Aryl, z.B. gegebenenfalls substituiertes Phenyl, wobei Substituenten u.a. Niederalkyl, Niederalkoxy oder Halogen sein können.

Niederalkylen ist z.B. 1,4-Butylen, 1,5-Pentylen oder 1,6-Hexylen.

Niederalkoxy ist insbesondere Methoxy oder Aethoxy, ferner n-Propoxy, Isopropoxy, n-Butoxy oder Isobutoxy, ferner n-Pentyloxy.

Halogen bedeutet vorzugsweise Halogen mit einer Atomnummer von höchstens 35, d.h. Fluor, Chlor oder Brom.

Es wird angenommen, dass die Verbindungen der vorliegenden Erfindung Prodrugs zur Hemmung der reversen Transkriptase sind, ein für Retroviren (oder Onconarviren), wie RNS-Tumor- und Leukämieviren, charakteristisches Enzym; Retroviren benötigen dieses Enzym für ihren natürlichen Replikationszyklus (Baltimore, Nature, Bd. 226, S. 1209 (1970); und Temin und Mizutani, Nature, Bd. 226, S. 1211 (1970)).

Die Wirkung auf Virusinfektionen kann anhand von in vivo-Testanordnungen festgestellt werden. So besitzen Verbindungen der Formel I in Tierexperimenten bei gewissen neoplastischen Krankheiten, die durch RNS-Tumorviren hervorgerufen werden, oder bei denen RNS-Tumorviren nachweisbar sind, eine starke Wirksamkeit. Sie verlängern z.B. die mittlere Ueberlebensdauer von Mäusen nach Infektion mit Rauscher-Leukämie-Virus (RLV, ein RNS-Tumorvirus).

Bei den Versuchen mit Rauscher-Leukämie 4 Q (NIH) werden weibliche Balb/c-Mäuse (28 bis 34 Tage alt) mit 0,2 ml einer zehnfach mit Hanks Lösung verdünnten Virussuspension aus Milzmaterial von 4-6 Wochen vorher infizierten Mäusen infiziert. In einem Versuch werden je 15 Tiere entweder mit der Testverbindung oder als Kontrollen mit Placebo kontinuierlich per os behandelt. Die erste Verabreichung erfolgt 30 Minuten vor der Infektion, weitere Verabreichungen täglich einmal und fünfmal in der Woche während 4 Wochen. Als Kriterium für die Wirkung dient die Verlängerung der Ueberlebenszeit. Während bei den Kontrolltieren ab 30 Tagen nach der Infektion eine Leukämie mit leukämischen Blutzellen von über $50000/mm^3$ gefunden werden können und die Tiere zu sterben beginnen, wird die Ueberlebenszeit der mit 125 mg/kg p.o. der Testsubstanz behandelten weiblichen Mäuse signifikant verlängert. Sie beträgt z.B. für das 6,6-Dimethyl-4,5-dihydro-6H-(1,3-dioxolo)[3,4]-naphtho[1,2-b]pyran bei 20-maliger p.o.-Verabreichung an weiblichen Balb/c-Mäusen von je 125 mg/kg (10 Tiere pro Gruppe) 60,9 Tage, verglichen mit Ueberlebungszeit der Kontrolltiere von 31,7 Tagen ($p \leq 0,001$ Cox-Test).

Die pharmakologische Wirksamkeit der Verbindungen der vorliegenden Erfindung ist begleitet von einer guten Verträglichkeit. So liegt die maximal tolerierte Dosis für das obengenannte 6,6-Dimethyl-4,5-dihydro-6H-(1,3-dioxolo)[3,4]naphtho[1,2-b]pyran in Mäusen nach einmaliger p.o- und i.p.-Verabreichung und anschliessender 7-tägiger Beobachtungsdauer über 2500 mg/kg bzw. über 1250 mg/kg.

Der Nachweis von Erkrankungen, die durch Retroviren verursacht werden, basiert z.B. auf den folgenden Befunden: Der Nachweis von Typ C-Retroviren und deren Bildung von reverser Transkriptase beim Menschen erfolgte zuerst bei einer T-Zellen Leukämie; das Virus wurde Human T-cell leukaemia virus (HTLV-I) genannt. Bei weiteren T-Zellen-Leukämien und -Lymphomen wurden die gleichen Viren und ein ähnliches Retrovirus (HTLV-II) gefunden (Gallo, Cancer Surveys (Ed. Franks, Wyke und Weiss), Bd. 3, S. 113-160 (Oxford Univ.

Press, 1984)). Schliesslich wurde ein derartiges Virus auch im Zusammenhang mit AIDS (Acquired Immune Deficiency Syndrome)-Erkrankungen isoliert (Gottlieb et al., Morbid. Mortal. Weekly Rep., Bd. 30, S. 25,(1981); Friedman-Kien et al., Morbid. Mortal. Weekly Rep., Bd. 30, S. 305 (1981); Gottlieb et al., New Engl. J. Med., Bd. 305, S. 1425 (1981); Masur, New Engl. J. Med., Bd. 305, S. 1431 (1981); Siegal et al., New Engl. J. Med., Bd. 305, S. 1439 (1981); CDC Task Force on Kaspoi's Sarcoma and Opportunistic Infections, New Engl. J. Med., Bd. 306, S. 248 (1982)); dieses wurde mit HTLV-III bezeichnet (Essex et al., Science, Bd. 220, S. 859 (1983), Gelmann et al., Bd. 220, S. 862 (1983); Gallo et al., Science, Bd. 220, S. 865 (1983); Gallo, Cancer Surverys, Bd. 3 S. 113 (1984); Barré-Sinoussi et al., Science Bd. 220, 868 (1983); Hirsch und Levy, Viruses in Human Malignancy and AIDS, ASCO/AACR Symposium, May 1984, Toronto).

Diese, der HTLV-Familie angehörenden Retroviren benötigen die reverse Transkriptase für die Bildung einer Doppelstrang-DNA (Provirus), welche in das Zell-Genom "integriert" wird und zur malignen Transformation führen kann (Strayer und Gillespie, The Nature and Organization of Retroviral Genes in Animal Cells. Virology Monographs, Bd. 17 (Springer Ed., 1980)).

Während nach der Induktion maligner leukämischer, lymphatischer oder tumoröser Neubildungen durch Retroviren eine Abnahme der reversen Transkriptase und der Viren HTLV-I und -II nachgewiesen werden kann und sekundäre, durch diese Viren induzierte Oncogene die Replikation der malignen Zellen steuern, ist das Virus HTLV-III und die reverse Transkriptase bei AIDS nicht nur im Frühstadium, sondern während des ganzen Krankheitsverlaufs nachweisbar. Die laufende Infektion und Funktionsstörung von immunkompetenten T-Helfer Zellen führt schliesslich zum Zusammenbruch des Immunsystems mit letalem Ausgang. Es ist anzunehmen, dass eine Hemmung der reversen Transkriptase und der Virusreplikation bei positivem Enzym- und Virusantigen-Nachweis (Beardsley, Nature, Bd. 311, S. 195 (1984)) in Risikopatienten, z.B.

Homosexuellen, den Krankheitsprozess beeinflusst. Bei der Induktion von Leukämien und Lymphomen durch Retroviren (HTLV-I und HTLV-II), wie möglicherweise auch bei durch Retroviren induzierten Sarkomen oder Mammakarzinomen, sollte dagegen eine prophylaktische Anwendung möglich sein, sofern eine leicht und breit verfügbare diagnostische Erfassung von derartigen Risikopatienten vorausgesetzt werden kann.

Auch im Zusammenhang mit der nicht-A- und nicht-B-Hepatitis ist die reverse Transkriptase als spezifisches Enzym in Assoziation mit Viruspartikeln gefunden worden (Seto et al., Lancet, S. 941 (1984)).

Die Verbindungen der vorliegenden Anmeldung können deshalb zur Prophylaxe und Therapie von Krankheiten, bei denen die reverse Transkriptase von Bedeutung ist, vor allem von durch Typ-C-Retroviren verursachten oder mitverursachten malignen Erkrankungen, aber auch von gewissen Immunkrankheiten und Autoimmunkrankheiten Verwendung finden. Als Tumorkrankheiten kommen in erster Linie durch Retroviren verursachte Leukämien, Lymphome und Lymphosarkome, sowie Osteosarkome und Mammakarzinome in Betracht. Die Verbindungen der Erfindung eignen sich besonders auch zur Rezidivprophylaxe nach chirurgischer Therapie, Strahlentherapie oder zytostatischer oder antimetabolischer Chemotherapie. Als Immunkrankheit sei AIDS, als Autoimmunkrankheit systemischer Lupus erythematosus genannt, bei denen nach neueren Befunden RNS-Tumorviren auch eine wichtige Rolle zu spielen scheinen (Dennman, Med., Biol., Bd. 53, S 61 (1975); Panem et al., New England J. Med., Bd. 295, 470 (1976)).

Die Erfindung betrifft insbesondere Verbindungen der Formel I, worin $R_1$ Wasserstoff oder Niederalkyl und $R_2$ Wasserstoff, Niederalkyl oder Phenyl bedeuten, $R_3$ und $R_4$ unabhängig voneinander Wasserstoff oder Niederalkyl bedeuten, und der Ring A keine weiteren Substituenten hat oder zusätzlich durch Niederalkyl, Niederalkoxy und/oder Halogen substituiert ist, wobei mit "nieder" bezeichnete Reste vorzugsweise 1 oder 2 Kohlenstoffatome enthalten, und Halogen eine Atomnummer von höchstens 35 hat.

Die Erfindung betrifft in erster Linie Verbindungen der Formel I, worin $R_1$ Wasserstoff oder Niederalkyl und $R_2$ Niederalkyl oder Phenyl bedeuten, und beide Reste $R_3$ und $R_4$ Wasserstoff oder unabhängig voneinander Wasserstoff oder Niederalkyl bedeuten, wobei mit "nieder" bezeichnete Reste vorzugsweise 1 oder 2 Kohlenstoffatome enthalten.

Die Verbindungen der Formel I können in an sich bekannter Weise hergestellt werden, z.B. indem man eine Verbindung der Formel

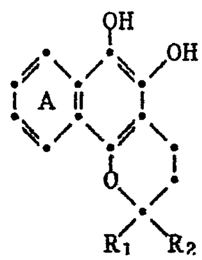

(II),

oder ein reaktionsfähiges Derivat davon mit einem den Rest der Formel

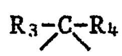

(Ia)

einführenden Reagens umsetzt.

Reaktionsfähige Derivate von Verbindungen der Formel II sind z.B. Salze, wie Alkalimetall-, z.B. Natrium- oder Kaliumsalze.

Reagenzien, die sich zur Einführung des Restes der Formel Ia eignen, sind z.B. Verbindungen der Formel

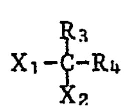

(III),

worin $X_1$ und $X_2$ unabhängig voneinander für veräthertes oder verestertes Hydroxy, in erster Linie jedoch zusammengenommen für die Diazogruppe stehen. Veräthertes Hydroxy ist z.B. Niederalkoxy, wie Methoxy oder Aethoxy, während verestertes Hydroxy insbesondere für Halogen, insbesondere Brom oder Jod, oder für Niederalkanoyloxy,

z.B. Acetoxy, steht. Bevorzugte Verbindungen der Formel III sind die entsprechenden Diazoverbindungen, insbesondere Diazoniederalkane, und in erster Linie Diazomethan.

Die Reaktion wird in an sich bekannter Weise durchgeführt, üblicherweise in Gegenwart eines geeigneten Verdünnungs- oder Lösungsmittels, das Diazoreagens vorzugsweise in Gegenwart eines wasserfreien ätherischen Lösungsmittels, wie Diäthyläther und/oder Tetrahydrofuran, wenn notwendig unter Kühlen oder Erwärmen, z.B. in einem Temperaturbereich von etwa -10°C bis etwa 100°C, in einem geschlossenen Gefäss und/oder in einer Inertgasatmosphäre.

Die Ausgangsstoffe der Formel II können z.B. durch Reduktion der entsprechenden bekannten Chinonverbindungen der Formel

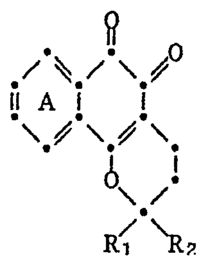

(IV),

wie durch katalytische Hydrierung, erhalten werden. Vorzugsweise werden die Ausgangsstoffe (II) in situ hergestellt, da sie sehr leicht in die entsprechenden Chinone der Formel IV zurückoxidiert werden. Da Diazoverbindungen der Formel III die Chinonverbindungen (IV) in die Ausgangsstoffe der Formel Ia reduzieren und gleichzeitig den gewünschten Rest der Formel IIa einführen können, stellt die Verfahrensvariante, lt. welcher eine Verbindung der Formel IV mit einer Verbindung der Formel III, worin $X_1$ und $X_2$ zusammen für die Diazogruppe stehen, insbesondere mit einem Diazoniederalkan und in erster Linie mit Diazomethan umgesetzt wird, wobei man mit einem Ueberschuss der Diazoverbindung (III) arbeitet, das bevorzugte Verfahren zur Herstellung von Verbindungen der Formel I dar.

Die vorliegende Erfindung betrifft ebenfalls pharmazeutische Präparate, die als Wirkstoffe eine der erfindungsgemässen Verbindungen der Formel I enthalten. Bei den erfindungsgemässen pharmazeutischen Präparaten handelt es sich um solche zur enteralen, wie oralen oder rektalen oder parenteralen Verabreichung, z.B. i.v., i.m. oder topisch. Die Präparate enthalten entweder ausschliesslich den Wirkstoff oder vorzugsweise den Wirkstoff vermischt mit einem pharmazeutisch anwendbaren Trägermaterial. Die Dosierung des Wirkstoffs hängt von der zu behandelnden Spezies, ihrem Alter und individuellen Zustand, sowie von der Applikationsweise ab.

Die pharmazeutischen Präparate enthalten von etwa 5 % bis etwa 95 % des Wirkstoffs, wobei einzeldosierte Präparate vorzugsweise von etwa 20 % bis etwa 90 % und nicht-einzeldosierte Präparate vorzugsweise etwa 5 % bis etwa 20 % Wirkstoff aufweisen. Erfindungsgemässe pharmazeutische Präparate in Dosiseinheitsform, wie Dragées, Tabletten, Kapseln, Suppositorien oder Ampullen, enthalten etwa 0,05 g bis etwa 1,5 g, vorzugsweise etwa 0,1 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls nach Zugabe von zusätzlichen Hilfsstoffen, zu Tabletten oder Dragées-Kernen verarbeitet.

Geeignete Trägerstoffe sind insbesondere bezüglich pharmakologischer Wirkung inerte Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose,

Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglycol.

Dragées-Kerne werden mit geeigneten, gegebenenfalls Magensaft-resistenten, Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragées-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigem Polyäthylenglycol, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner

kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffine, Polyäthylenglykole oder höhere Alkanole. Ferner können auch Gelatine-Rektalkapseln verwendet werden, die aus einer Kombination des Wirkstoffes mit einer Grundmasse bestehen; als Grundmassenstoffe kommen z.B. flüssige Triglyceride, Polyäthylenglykole oder Paraffine in Frage.

Zur parenteralen Verabreichung kommen z.B. Suspensionen von Verbindungen der vorliegenden Erfindung wie entsprechende ölige Injektionssuspensionen, in Betracht, wobei man geeignete lipophile Lösungsmittel oder Vehikel, wie fette Oele, z.B. Sesamöl, oder synthetische Fettsäureester, z.B. Aethyloleat oder Triglyceride, verwendet, oder es eignen sich wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Zur parenteralen Verabreichung eignen sich insbesondere Liposomendispersionen mit Verbindungen der Formel I. Die Liposomendispersionen werden aus mindestens zwei Lipidkomponenten, z.B. Phosphatidylserin und Phosphatidyläthanolamin oder Phosphatidylserin und Phosphatidylcholin, gebildet, welche Verbindungen (I) verkapseln.

Zur Herstellung der Liposomendispersion eignet sich für die eine Lipidkomponente Phosphatidylserin natürlicher Herkunft mit verschiedenen oder identischen $C_{10}$-$C_{20}$-Alkanoylresten, z.B. n-Dodecanoyl, n-Tetradecanoyl, n-Hexadecanoyl oder n-Octadecanoyl, oder $C_{10}$-$C_{20}$-Alkenoylresten, z.B. 9-cis-Dodecenoyl, 9-cis-Tetradecenoyl, 9-cis-Hexadecenoyl, 6-cis-, 6-trans-, 9-cis-, 9-trans- oder 11-cis-Octadecenoyl oder 9-cis-Icosenoyl, z.B. Phosphatidylserin aus dem Rinderhirn, oder synthethisches Phosphatidiylserin mit identischen $C_{10}$-$C_{20}$-Alkenoylresten, z.B. Natrium-1,2-di-(9-cis-octadecenoyl)-3-sn-phosphatidyl-(S)-serin, und für die andere Lipidkomponente Phosphatidylcholin natürlicher Herkunft mit verschiedenen oder identischen $C_{10}$-$C_{20}$-Alkanoylresten, z.B. Phosphatidylcholin aus dem Hühnerei oder aus Sojaöl, synthetisches Phosphatidylcholin mit identischen $C_{10}$-$C_{20}$-Alkanoylresten, z.B. Dimyristoyl-, Distearoyl- oder Dipalmitoylphosphatidiylcholin, oder synthetisches Phosphatidylcholin mit einem $C_{10}$-$C_{20}$-Alkanoyl- und einem $C_{10}$-$C_{20}$-Alkenoylrest, z.B. 1-n-Hexadecanoyl-2-(9-cis-octadecenoyl)-3-sn-phosphatidiylcholin.

Die Liposomendispersion lässt sich herstellen, indem man beispielsweise ein Lyophilisat aus den Lipidkomponenten und der Verbindung (I) gemäss dem in der Deutschen Offenlegungsschrift 2818655 beschriebenen Verfahren herstellt, vorzugsweise nach Lösen der Lipidkomponenten und des Wirkstoffs in tert-Butanol, und Abziehen des Lösungsmittels bei Temperaturen unterhalb -20°, und dieses Lyophilisat in einer isotonischen Pufferlösung dispergiert. Die Dispersion erfolgt durch Schütteln (Vortex-Mischer) oder Rühren der wässrigen Phase. Die Liposomendispersion kann anschliessend durch Zentrifugation angereichert und/oder durch Gelfiltration oder Extrusion durch geradporige Filter abgetrennt werden.

Dieses Verfahren eignet sich, wenn lipophile bzw. in organischen Lösungsmitteln, z.B. tert-Butanol, lösliche Verbindungen (I) verwendet werden. Bei wasserlöslichen Verbindungen (I) stellt man ein Lyophilisat nur aus den Lipidkomponenten her und dispergiert dieses in wässriger Phase, welche die gelöste Verbindung (I) enthält.

Die Erfindung betrifft ebenfalls ein Verfahren zur Behandlung von Krankheiten, bei denen, wie oben beschrieben worden ist, die reverse Transkriptase von Bedeutung ist, dadurch gekennzeichnet, dass man eine prophylaktisch oder therapeutisch wirksame Menge einer erfindungsgemässen Verbindung der Formel I verabreicht. Dabei verwendet man in erster Linie die obengenannten pharmazeutischen Präparate, wobei man einem Warmblüter von etwa 70 kg Gewicht eine tägliche Dosis von etwa 0,1 g bis etwa 5 g, vorzugsweise von etwa 0,5 g bis etwa 1,5 g, einer Verbindung der vorliegenden Erfindung verabreicht.

Die nachfolgenden Beispiele illustrieren die vorliegende Erfindung; Temperaturen werden in Celsiusgraden angegeben.

Beispiel 1: 6,6-Dimethyl-4,5-dihydro-6H-(1,3-dioxolo)[3,4]-naphtho[1,2-b]pyran

Eine Lösung von Diazomethan in Diäthyläther (hergestellt aus 40 g Nitrosomethylharnstoff und 400 ml Diäthyläther) wird tropfenweise bei einer Temperatur von 2-3° mit einer Lösung von 22,4 g 2,2-Dimethyl-3,4-dihydro-2H-naphtho[1,2-b]pyran-5,6-dion (ß-Lapachon) in 200 ml Tetrahydrofuran versetzt. Das Reaktionsgemisch wird während 16 Stunden bei Raumtemperatur stehen gelassen. Das Lösungsmittelgemisch wird unter vermindertem Druck abgedampft, der Rückstand in 400 ml Methylenchlorid gelöst und rasch durch 400 g Silicagel (Merck; 0.063 - 0,2 mm) filtriert. Aus den ersten Filterfraktionen wird beigefarbenes, kristallines Material erhalten (Dünnschichtchromatogramm auf Silicagel 60 $F_{254}$ Merck; Rf 0,73 (Chloroform)), das, aus Isopropanol umkristallisiert die Titelverbindung ergibt, Smp. 84-85°.

Beispiel 2: 6,6-Dimethyl-4,5-dihydro-6H-(1,3-dioxolo)[3,4]-naphtho[1,2-b]pyran

Eine Lösung von 12,1 g 2,2-Dimethyl-3,4-dihydro-2H-naphtho-[1,2-b]pyran-5,6-dion (β-Lapachon) in 120 ml Dimethylformamid wird mit 11 g Triäthylamin und 1 g eines Platin-auf-Kohle-Katalysators (5 %) versetzt und bei Raumtemperatur und unter normalem Druck hydriert. Unter Ausschluss von Sauerstoff werden 10,5 ml Methyleniodid zugegeben und das Gemisch während 5 Stunden bei 100° erwärmt. Man kühlt ab, filtriert und engt das Filtrat unter vermindertem Druck auf ein Volumen von etwa 40 ml ein. Man verdünnt mit 200 ml

Wasser; der entstandene Niederschlag wird abfiltriert und mit Diäthyläther verrührt. Die überstehende Lösung wird abdekantiert, mit Wasser gewaschen, über Natriumsulfat getrocknet und unter vermindertem Druck eingedampft. Der Rückstand wird in Methylenchlorid aufgenommen und an 200 g Silicagel unter Verwendung von Methylenchlorid chromatographiert. Die gewünschte Titelverbindung wird aus den ersten Fraktionen erhalten; es ist lt. Dünnschichtchromatogramm mit der Titelverbindung des Beispiels 1 identisch.

Beispiel 3: 9-Chlor-6,6-dimethyl-4,5-dihydro-6H-(1,3-dioxolo)-[3,4]naphtho[1,2-b]pyran

Eine auf +5° gekühlte Lösung von 500 mg 9-Chlor-2,2-dimethyl-3,4-dihydro-2H-naphtho[1,2-b]pyran-5,6-dion (9-Chlor-β-Lapachon), Herstellung gemäss J.Med.Chem. 1984, 27, 990-994, in 5 ml THF wird mit 20 ml einer Lösung von Diazomethan in Aether versetzt und während 16 Stunden bei Raumtemperatur stehen gelassen. Das Lösungsmittelgemisch wird bei reduziertem Druck entfernt und der Rückstand aus 4 ml Isopropanol kristallisiert. Es werden gelb-beige Kristalle der Titelverbindung vom Schmelzpunkt 116-118° erhalten. Aus der Mutterlauge wird durch Chromatographieren an 50 g Silicagel und Elution mit Essigester-Cyclohexan (1:3) weitere Titelverbindung erhalten.

Beispiel 4: 6-Methyl-6-phenyl-4,5-dihydro-6H-(1,3-dioxolo)[3,4]-naphtho[1,2-b]pyran

Eine auf +5° gekühlte Lösung von 500 mg 2-Methyl-2-phenyl-3,4-dihydro-2H-naphtho[1,2-b]pyran (2-Desmethyl-2-phenyl-β-Lapachon), Herstellung gemäss J.Med.Chem. 1984, 27, 994 in 5 ml THF wird mit 20 ml einer Lösung von Diazomethan in Aether versetzt und während 16 Stunden bei Raumtemperatur stehen gelassen. Das Lösungsmittelgemisch wird bei reduziertem Druck entfernt und der Rückstand an 50 g Silicagel und durch Elution mit Aethylenchlorid-Methanol (98:2) chromatographiert. Aus Isopropanol wird gelb-beige Titelverbindung erhalten.

Beispiel 5: Tabletten, enthaltend 500 mg 6,6-Dimethyl-4,5-dihydro-2H-(1,3-dioxolo)[3,4]naphtho[1,2-b]pyran, können wie folgt hergestellt werden:

Zusammensetzung (für 10000 Tabletten):

| | |
|---|---|
| 6,6-Dimethyl-4,5-dihydro-2H-(1,3-dioxolo)-[3,4]naphtho[1,2-b]pyran | 5000,00 g |
| Weizenstärke | 790,00 g |
| Stearinsäure | 30,00 g |
| Magnesiumstearat | 30,00 g |
| Talk | 400,00 g |

Der Wirkstoff wird mit verkleisterter Weizenstärke, die durch Anrühren von 500 g Weizenstärke mit etwa 1300 ml entmineralisiertem Wasser erhalten wird, und mit weiteren 600 ml entmineralisiertem Wasser gleichmässig befeuchtet, zu einer schwach plastischen Masse geknetet und durch ein Sieb mit einer Maschenweite von etwa 3 mm getrieben. Das Granulat wird anschliessend getrocknet und erneut durch ein Sieb geschlagen. Dem trockenen und auf eine einheitliche Korngrösse gebrachten Granulat werden das Magnesiumstearat, die Stearinsäure, der Talk und der Rest der Weizenstärke zugemischt und das erhaltene Gemisch wird zu Tabletten verpresst.

Beispiel 6: Eine 10 %ige Suspension enthaltend 6,6-Dimethyl-4,5-dihydro-2H-(1,3-dioxolo)[3,4]naphtho[1,2-b]pyran, kann wie folgt hergestellt werden:

Zusammensetzung (für 5000 ml):

| | |
|---|---|
| 6,6-Dimethyl-4,5-dihydro-2H-(1,3-dioxolo)-[3,4]naphtho[1,2-b]pyran | 500,00 g |
| Propylenglycol | 500,00 g |
| Hydroxypropylmethylcellulose | 25,00 g |
| Zitronensäure | 5,00 g |
| Saccharin-natrium | 2,50 g |
| 4-Hydroxy-benzoesäuremethylester | 6,00 g |

| | | |
|---|---|---|
| 4-Hydroxy-benzoesäurepropylester | 1,50 | g |
| Himbeeraroma | 5,00 | g |
| 70 %ige wässrige Sorbitlösung | 1250 | ml |
| entmineralisiertes Wasser | q.s. | |

Eine Suspension des Wirkstoffs in 250 g Propylenglycol und 1250 ml entmineralisiertem Wasser wird mit einer Kolloidmühle gemahlen. Die feine Suspension, mit einer durchschnittlichen Teilchengrösse von unter 10 Âm, wird in einer Lösung der Hydroxypropylmethylcellulose, der Zitronensäure und des Saccharin-natriums in 1250 ml entmineralisiertem Wasser und in der Sorbit-Lösung dispergiert. Unter Rühren werden zu dieser Suspension eine Lösung des 4-Hydroxy-benzoesäuremethylesters und des 4-Hydroxybenzoesäurepropylesters in 250 g Propylenglycol und das Himbeeraroma gegeben. Nach dem Ergänzen mit entmineralisiertem Wasser auf das Volumen von 5000 ml, erhält man eine Suspension, die 500 mg des Wirkstoffs in 5 ml enthält.

Beispiel 7: Kapseln, enthaltend je 300 mg 6,6-Dimethyl-4,5-dihydro-2H-(1,3-dioxolo)[3,4]naphtho[1,2-b]pyran, können wie folgt hergestellt werden:

Zusammensetzung (für 10000 Kapseln)

| | | |
|---|---|---|
| 6,6-Dimethyl-4,5-dihydro-2H-(1,3-dioxolo)-[3,4]naphtho[1,2-b]pyran | 3000,00 | g |
| Magnesiumstearat | 100,00 | g |

Der Wirkstoff wird mit dem Magnesiumstearat vermischt und je 0,31 g des Gemisches mit Hilfe einer Verkapselungsmaschine in Hartgelatinekapseln abgefüllt.

Beispiel 8: Eine wässrige Injektionssuspension (für intramuskuläre Verabreichung), enthaltend 1,0 g 6,6-Dimethyl-4,5-dihydro-2H-(1,3-dioxolo)[3,4]naphtho[1,2-b]pyran, pro 5 ml, kann wie folgt hergestellt werden:

Suspensionsmedium:

| | | |
|---|---|---|
| Natriumsalz von Carboxymethylcellulose | | 0,10 % |
| Gemisch von Natriumdihydrogenphosphat und Dinatriumhydrogenphosphat | | 0,15 % |
| Natriumchlorid | | 0,75 % |
| Natriumsalz der S-Aethylmercuri-thiosalicylsäure (Thiomersal) | | 0,02 % |
| 1,2 Propylenglycol | | 20,00 % |
| Destilliertes Wasser | ad | 100,00 % |

Der sterile Wirkstoff wird unter antimikrobiellen Bedingungen mit dem Suspensionsmedium so verarbeitet, dass eine sterile Suspension erhalten wird, die 1,0 g des Wirkstoffs in 5 ml enthält.

Beispiel 9: Eine wässrige Injektionssuspension (für intramuskuläre Verabreichung), enthaltend 0,75 g 6,6-Dimethyl-4,5-dihydro-2H-(1,3-dioxolo)[3,4]naphtho[1,2-b]pyran pro 5 ml, kann wie folgt hergestellt werden:

Suspensionsmedium:

| | |
|---|---|
| Natriumchlorid | 0,90 % |
| Natriumsalz der S-Aethylmercuri-thiosalicylsäure (Thiomersal) | 0,02 % |
| Polyoxyäthylen(20)Sorbitan Monostearat (Molekulargewicht: 1311,7 g; Tween 60®) | 0,75 % |
| Natriumsalz von Carboxymethylcellulose | 0,50 % |

Der sterile Wirkstoff wird unter antimikrobiellen Bedingungen mit dem Suspensionsmedium so verarbeitet, dass man eine sterile Suspension erhält, die 0,75 g Wirkstoff in 5 ml enthält.

Patentansprüche (für alle benannten Vertragsstaaten ausser Oesterreich)

1. Verbindungen der Formel

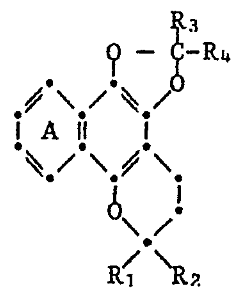

(I),

worin $R_1$ und $R_2$ unabhängig voneinander Wasserstoff, Niederalkyl, Aryl oder zusammengenommen Niederalkylen bedeuten, $R_3$ und $R_4$ unabhängig voneineander Wasserstoff, Niederalkyl, Aryl oder zusammengenommen Niederalkylen bedeuten, und der Ring A keine zusätzliche Substituenten hat oder zusätzlich durch Niederalkyl, Niederalkoxy und/oder Halogen substituiert ist.

2. Verbindungen der Formel I gemäss Anspruch 1, worin $R_1$ Wasserstoff oder Niederalkyl und $R_2$ Wasserstoff, Niederalkyl oder Phenyl bedeuten, $R_3$ und $R_4$ unabhängig voneinander Wasserstoff oder Niederalkyl bedeuten, und der Ring A keine zusätzlichen Substituenten hat oder zusätzlich durch Niederalkyl, Niederalkoxy und/oder Halogen substituiert ist, wobei mit "nieder" bezeichnete Reste vorzugsweise 1 oder 2 Kohlenstoffatome enthalten, und Halogen eine Atomnummer von höchstens 35 hat.

3. Verbindungen der Formel I gemäss Anspruch 1, worin $R_1$ Wasserstoff oder Niederalkyl, $R_2$ Niederalkyl oder Phenyl und beide Reste $R_3$ und $R_4$ Wasserstoff oder unabhängig voneinander Wasserstoff oder Niederalkyl bedeuten, wobei mit "nieder" bezeichnete Reste vorzugsweise 1 oder 2 Kohlenstoffatome enthalten.

4. 6,6-Dimethyl-4,5-dihydro-6H-(1,3-dioxolo)|3,4|naphtho[1,2-b]-pyran, gemäss Anspruch 1.

5. Pharmazeutische Präparate enthaltend als Wirkstoff eine Verbindung der Formel I gemäss einem der Ansprüche 1 bis 4.

6. Verbindungen der Formel I zur Anwendung in einem Verfahren zur therapeutischen Behandlung des menschlichen oder tierischen Körpers.

7. Die Verbindungen der Formel I gemäss einem der Ansprüche 1 bis 4 als Hemmer der reversen Transkriptase.

8. Verwendung der Verbindungen der Ansprüche 1 bis 4 zur Herstellung von pharmazeutischen Präparaten.

9. Verfahren zur Herstellung von Verbindungen des Anspruchs 1, dadurch gekennzeichnet, dass man eine Verbindung der Formel

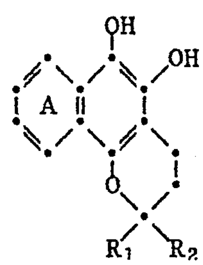

(II),

oder ein reaktionsfähiges Derivat davon mit einem, den Rest der Formel

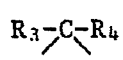

(Ia)

einführenden Reagens umsetzt.

10. Die nach dem Verfahren des Anspruchs 9 erhältlichen Verbindungen.

FO 7.4 RS/sm*

Ansprüche (für den Vertragsstaat Oesterreich)

1. Verfahren zur Herstellung von Verbindungen der Formel

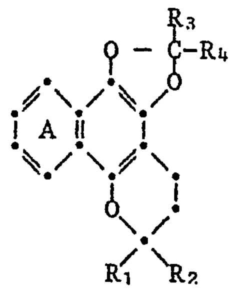

(I),

worin $R_1$ und $R_2$ unabhängig voneinander Wasserstoff, Niederalkyl, Aryl oder zusammengenommen Niederalkylen bedeuten, $R_3$ und $R_4$ unabhängig voneinander Wasserstoff, Niederalkyl, Aryl oder zusammengenommen Niederalkylen bedeuten, und der Ring A keine zusätzliche Substituenten hat oder zusätzlich durch Niederalkyl, Niederalkoxy und/oder Halogen substituiert ist, dadurch gekennzeichnet, dass man eine Verbindung der Formel

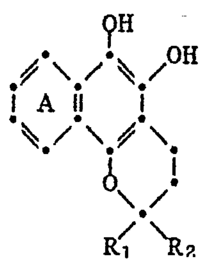

(II),

oder ein reaktionsfähiges Derivat davon mit einem, den Rest der Formel

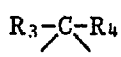

(Ia)

einführenden Reagens umsetzt.

2. Verfahren zur Herstellung von Verbindungen der Formel I gemäss Anspruch 1, worin $R_1$ Wasserstoff oder Niederalkyl und $R_2$ Wasserstoff, Niederalkyl oder Phenyl bedeuten, $R_3$ und $R_4$ unabhängig voneinander Wasserstoff oder Niederalkyl bedeuten, und der Ring A

keine zusätzlichen Substituenten hat oder zusätzlich durch Niederalkyl, Niederalkoxy und/oder Halogen substituiert ist, wobei mit "nieder" bezeichnete Reste vorzugsweise 1 oder 2 Kohlenstoffatome enthalten, und Halogen eine Atomnummer von höchstens 35 hat, dadurch gekennzeichnet, dass man ein entsprechend substituiertes Ausgangsmaterial der Formel II mit dem entsprechenden, den Rest der Formel Ia einführenden Reagens umsetzt.

3. Verfahren zur Herstellung von Verbindungen der Formel I gemäss Anspruch 1, worin $R_1$ Wasserstoff oder Niederalkyl, $R_2$ Niederalkyl oder Phenyl und beide Reste $R_3$ und $R_4$ Wasserstoff oder unabhängig voneinander Wasserstoff oder Niederalkyl bedeuten, wobei mit "nieder" bezeichnete Reste vorzugsweise 1 oder 2 Kohlenstoffatome enthalten, dadurch gekennzeichnet, dass man ein entsprechend substituiertes Ausgangsmaterial der Formel II mit dem entsprechenden, den Rest der Formel Ia einführenden Reagens umsetzt.

4. Verfahren zur Herstellung von 6,6-Dimethyl-4,5-dihydro-6H-(1,3-dioxolo)[3,4]naphtho[1,2-b]pyran, gemäss Anspruch 1, dadurch gekennzeichnet, dass man β-Lapachon mit Diazomethan umsetzt.

FO 7.4 RS/sm*

Europäisches Patentamt

# EUROPÄISCHER RECHERCHENBERICHT

0184990

Nummer der Anmeldung

EP 85810556.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | GB - A - 1 040 176 (MERCK & CO)<br>---- | | C 07 D 493/04<br>A 61 K 31/35 |
| | | | RECHERCHIERTE SACHGEBIETE (Int Cl 4) |
| | | | C 07 D 493/00 |

Der vorliegende Recherchenbericht wurde für alle Patentansprüche erstellt.

| Recherchenort | Abschlußdatum der Recherche | Prüfer |
|---|---|---|
| WIEN | 03-03-1986 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82